# FASCICULE DE BREVET EUROPEEN

(11) **EP 1 283 907 B1**
(45) Date de publication et mention de la délivrance du brevet: **08.09.2004**
(21) Numéro de dépôt: 01928158.3
(22) Date de dépôt: 16.05.2001
(51) Int. Cl.: C12Q 1/68, G01N 33/574

(54) **METHODE POUR LE DIAGNOSTIC DE CANCERS**
VERFAHREN ZUR DIAGNOSE VON KREBS
CANCER DIAGNOSIS METHOD

(30) Priorité: 26.05.2000 EP 00111370
(43) Date de publication de la demande: 19.02.2003
(73) Titulaire: Chen, Xu Qi, University of Teaxs, Laboratoire de Leucémie, Houston, Texas 77030 (US); Stroun, Maurice, 1208 Geneve (CH); Anker, Philippe, 1203 Geneve (CH)
(72) Inventeur: Chen, Xu Qi, University of Teaxs, Laboratoire de Leucémie, Houston, Texas 77030 (US); Stroun, Maurice, 1208 Geneve (CH); Anker, Philippe, 1203 Geneve (CH)
(74) Mandataire: Micheli & Cie
(86) Numéro de dépôt international: PCT/IB2001/000852
(87) Numéro de publication internationale: WO 2001/090409

(56) Documents cités:
- EP-A- 0 926 245
- EP-A- 0 990 701
- WO-A-97/18322
- WO-A-98/28442
- WO-A-99/41406
- YAJIMA T ET AL: "Establishment of quantitative reverse transcription-polymerase chain reaction assays for human telomerase-associated genes" CLINICA CHIMICA ACTA, vol. 290, janvier 2000 (2000-01), pages 117-127, XP001016319
- KOK J ET AL: "Real-time quantification of human telomerase reverse transcriptase mRNA in tumors and healthy tissues" CLINICAL CHEMISTRY, vol. 46, no. 3, mars 2000 (2000-03), pages 313-18, XP002176598
- ANKER P: "Quantitative aspects of plasma/serum DNA in cancer patients" ANNALS OF THE NEW YORK ACADEMY OF SCIENCES, vol. 906, avril 2000 (2000-04), pages 5-7, XP001016322

## Description

La présente invention concerne une méthode de diagnostic et/ou de suivi de l'évolution de divers types de cancers, par exemple après un traitement de chimiothérapie ou après une opération.

On sait que le diagnostic et le suivi de l'évolution des cancers sont effectués, à part l'observation et l'examen direct de tumeurs, par analyse de biopsies ou, dans le cas de cancers du sang, de la moelle osseuse, ce qui implique soit une intervention chirurgicale, soit un test Invasif du type biopsie ou aspiration médullaire. Or, en plus du caractère désagréable, voire dangereux, pour les patients de telles méthodes, il a été constaté qu'elles pouvaient en outre être peu précises. En ce qui concerne le cancer du sein, de grands efforts sont faits pour développer le test de détection par mammographie. Bien que plusieurs études indiquent que la mammographie de masse peut être une stratégie utile pour réduire la mortalité due au cancer du sein, cette méthode comporte un certain nombre de désavantages. Parmi ceux-ci, il faut relever un fort taux de faux positifs, des faux négatifs fréquents et d'énormes dépenses pour l'Etat. Ainsi, lorsque les avantages et les désavantages sont comparés, il n'est pas surprenant que cette forme d'examen ait engendré des débats contradictoires animés depuis vingt ans.

Le but de cette invention consiste donc à fournir une méthode de diagnostic et/ou de suivi de l'évolution de divers types de cancers qui soit d'une part plus précise et plus fiable, et d'autre part qui soit plus facile à réaliser et n'impliquant pas de test invasif sur les patients.

La méthode de diagnostic et/ou suivi de l'évolution de cancers, objet de l'invention et visant à atteindre le but précité, comprend l'analyse du composant ribonucléique (ARN) dans le plasma ou le sérum sanguin.

La télomérase est une enzyme de nature ribonucléoprotéique qui synthétise des séquences répétées télomériques sur les terminaisons des chromosomes. Les téloméres protègent les extrémités des chromosomes, et à chaque division cellulaire les télomères se raccourcissent. La télomérase utilise pour synthétiser ces télomères un ou deux segments de son composant ARN en tant que matrice.

L'activité de cette enzyme est devenue un indicateur établi pour le diagnostic et le pronostic de la plupart des tumeurs cancéreuses. L'expression de l'ARN de télomérase humaine (hTR) ou de l'enzyme de transcriptase inverse de l'ARN télomérase (hTERT) ou de la protéine associée (TEP 1) a été déterminée lors de la progression de plusieurs espèces de tumeurs, ce qui a permis d'établir une corrélation entre cette expression (la quantité d'ARN) et l'activité de (a télomérase. La plupart des cancers et des lignées cellulaires immortalisées ont une forte activité télomérasique, reflet d'un mécanisme échappant aux régulations normales de vieillissement.

Or, et bien que les composant ARN de la télomérase soient des composants cellulaires, il a été constaté que, de manière surprenante, ces composants se retrouvaient également sous forme extra-cellulaire dans le plasma ou le sérum sanguin.

En effet, les présents inventeurs ont mis en évidence la présence de hTR, de hTERT et de TEP 1 dans le plasma ou le sérum sanguin de personnes souffrant notamment de cancers du sein, de l'ovaire, de l'estomac ou du colon, alors que ces produits sont absents dans le sang de personnes saines. Contrairement aux divers marqueurs d'acide désoxyribonucléique (ADN) déjà retrouvés dans le plasma ou le sérum, tels les mutations du gène ras ou p53 ou les nombreux microsatellites de la littérature, les composants ARN de la télomérase semblent pouvoir être employés pour toutes les sortes de tumeurs. Il s'agit d'un marqueur plasmatique ou séreux général pour le cancer. Ainsi pour la plupart des cancers, le taux de sérums porteurs d'ARN de la télomérase est plus important que celui obtenu par les autres marqueurs d'acides nucléiques employés jusqu'à maintenant.

Plus particulièrement, la méthode de diagnostic selon l'invention consiste à extraire l'ARN du plasma ou du sérum sanguin, à purifier et à amplifier cet ARN, puis à établir la présence et optionnellement la quantité de produit de réaction polymérase en chaîne reverse (RT PCR) représentant les composants hTR, hTERT ou du TEP 1 ceci de manière comparative entre le plasma ou le sérum d'une personne présumée malade et celui d'une personne en bonne santé.

Les produits d'amplification PCR des composants ARN transcrits en ADN grâce à la RT PCR sont mis en évidence et le cas échéant quantifiés par exemple en utilisant des méthodes à coloration sur gel. Cette analyse peut également se faire différemment sur d'autres gels ou sans gel par technique immunologique radioactive (RIA), par ELISA (Enzyme Linked Immunosorbent Assay ou par test microchips (gene array). Une amplification quantitative sur Taq Man (Perkin Elmer Biosystem) ou sur capillaires Light Cycler (Hofmann-La Roche) améliore la précision de la détection du hTR, du hTERT et du TEP 1.

De même, toute technique d'extraction, de purification et d'amplification de l'ARN dans le plasma ou le sérum peut être employée.

La présente invention sera maintenant illustrée de manière non limitative par l'Exemple qui suit, relatif au diagnostic des cancers du sein. Toutefois, il convient de constater que la portée de la présente invention n'est en aucune manière limitée au diagnostic de ce type de cancer. Des tests préliminaires ont en effet démontré également la présence de hTR, hTERT et TEP 1 dans le sérum de patients atteints notamment de cancers ovariens ou gastro-intestinaux. On peut en conclure d'ores et déjà que les hTR, hTERT et TEP 1 du plasma ou du sérum sont à même de constituer des marqueurs généraux de nombreux types de cancers.

### Exemple

Diagnostic du cancer du sein par détection de hTR, de hTERT ou de TEP 1 dans le plasma ou le sérum sanguin.

Des échantillons de sang (2-3 ml) ont été prélevés préopérativement sur 18 patientes, porteuses de petites tumeurs cancéreuses du sein (T1 ou T2) encore différenciées (G1 ou G2) et en principe sans nodules cancéreux ni métastases. Les échantillons ont ensuite été centrifugés à 900 g pendant 15 minutes à température ambiante et le sérum recueilli. Une seconde centrifugation à 900g pendant 15 min. a été effectuée pour se débarrasser de tous débris. Les échantillons de sérum ont été conservés à -70°C.

L'extraction de l'ARN a été effectuée en utilisant une technique usuelle et un kit commercial ("SV Total RNA Isolation System" de la société Promega WI, USA), selon les instructions du fournisseur. Par rapport à ces instructions une modification a été apportée, consistant plus particulièrement en l'adjonction de 175 µl de tampon "SVRNA Lysis" directement à 100µl de sérum ou de plasma frais (ou du moins n'ayant pas été dégélé plus d'une fois).

Détection de hTR, de hTERT, de TEP 1 et du rRNA (RNA de référence) au moyen de la PCR par transcriptase réverse (RT-PCR) :

Pour la détection de la présence et de la quantité de hTR et de rRNA, on a utilisé un kit Qiagen (One Step RT-PCR). 1 mg d'ARN de sérum a été introduit dans un mélange réactionnel de 25 µl RT-PCR contenant 400µM de chaque dNTP, omniscript^{TM} reverse transcriptase, sensiscript^{TM} reverse transcriptase, hot-start Taq^{TM} DNA polymerase et 0,15µM d'amorce (primer) à savoir pour hTR (sens) GAAGGGCGTAGGCGCCGTGCTTTTGC et (antisens) GTTTGCTCTAGAATGAACGGTGGAAGG. Le mélange a été incubé à 50°C pendant au moins 30 min. pour la transcription reverse, puis à 95°C pendant 15 min. pour activer la "hot-start Taq^{TM} DNA Polymerase", et 50 cycles à 94°C pendant 30 sec., à 65°C pendant 1 min., à 72°C pendant 1 min., avec une extension finale à 72°C pendant 10 min.

Les mêmes conditions de RT-PCR ont été utilisées pour la détection de hTERT, avec toutefois une concentration d'amorce de 0,3 µM, les amorces étant les suivantes : (sens) TGACACCTCACCTCACCCAC et (antisens) CACTGTCTTCCGCAAGTTCAC. Les mêmes conditions ont été utilisées pour la détection du TEP 1, les amorces étant les suivantes :

(sens) CACCTGCGACGATATTTCT et (antisens)

CGAGGGTTGTACTTAGCCA (concentrations amorce 0,3μM).

En ce qui concerne le RNA de contrôle, le GAPDH et les mêmes conditions que pour hTR ont également été employées, avec une concentration d'amorce de 0,3 µM; les séquences de l'amorce GAPDH étant les suivantes : (sens) CGGAGTCAACGGATTTGGTCGTAT et (antisens) AGCCTTCTCCATGGTGGTGAAGAC.

Toutes les séquences d'acides aminés mentionnées ci-dessus à titre d'exemples d'amorces sont connues en tant que telles, et peuvent par exemple être consultées sur le site Internet de la Genome Database (http://www.gdb.org/).

La PCR a donné un produit d'amplification de 111bp pour le hTR, de 95 bp pour le hTERT de 150 bp pour le TEP 1 et de 306 bp pour GAPDH du RNA de contrôle. Ce résultat a été analysé à 55°C sur gels "Elchrom Scientific S-50" (Elchrom Scientific, Cham, Suisse) , coloré au "SYBR-gold" (Molecular Probe, Eugene, OR, USA) pendant 45 min., décoloré 2 fois pendant 30 min. dans de l'eau déionisée à température ambiante et dans l'obscurité.

### Résultats :

En mettant en oeuvre la technique décrite ci-dessus, il en résulte qu'il est effectivement possible de détecter un produit PCR issu de l'ARN de référence dans tous les échantillons analysés. Quantitativement, tous les échantillons sont semblables, qu'ils proviennent du sérum d'une personne saine ou de celui d'une personne malade.

En ce qui concerne le produit d'amplification obtenu avec les amorces spécifiques de l'hTR, seul l'ARN provenant de patientes souffrant d'un cancer du sein s'est révélé positif dans 25% des cas. Quant à l'ARN de hTERT, il a été détecté dans 22% des sérums des patientes malades. En ce qui concerne le TEP 1, il est délectable dans 20% des cas. La combinaison des trois marqueurs a donc permis de détecter un cancer du sein dans 55% des cas; ce taux est clairement supérieur à celui que l'on peut obtenir avec les techniques actuellement connues, lequel dépasse rarement environ 30%. Par contre, les ARN de la télomérase sont absents des sérums de contrôle (personnes saines).

## Revendications

1. Méthode pour le diagnostic et/ou le suivi de l'évolution de cancers comprenant l'analyse d'ARN de l'enzyme télomérase présents dans le plasma ou le sérum sanguin, **caractérisée par le fait qu'**on extrait de l'ARN du plasma ou du sérum, qu'on purifie et qu'on amplifie cet ARN, puis qu'on analyse l'ARN hTERT codant pour la partie catalytique de l'enzyme ou l'ARN TEP 1 codant pour la protéine associée.

2. Méthode selon la revendication 1, **caractérisée par le fait que** l'ARN est amplifié par la technique de transcriptase reverse en chaîne (RT-PCR).

3. Méthode selon la revendication 2, **caractérisée par le fait que** lesdits ARN sont analysés par coloration sur gels, par technique immunologique radioactive (RIA), par test enzyme linked immunosorbent (ELISA) ou par test microchips (gene array).

4. Méthode selon la revendication 3, **caractérisée par le fait que** les ARN sont soumis à une quantification.

5. Méthode selon la revendication 3 ou 4, **caractérisée par le fait que** la quantification est effectuée sur « Taq Man » ou sur capillaires « Light Cycler ».

## Patentansprüche

1. Verfahren zur Diagnose und/oder Überwachung der Entwicklung von Krebs, die Analyse von im Blutplasma oder -serum vorliegenden RNA des Enzyms Telomerase umfassend, **dadurch gekennzeichnet, dass** man RNA aus dem Plasma oder Serum extrahiert, diese RNA reinigt und amplifiziert, dann die für den katalytischen Abschnitt des Enzyms kodierende hTERT-RNA oder die für das assoziierte Protein kodierende TEP1-RNA analysiert.

2. Verfahren nach Anspruch 1, **dadurch gekennzeichnet, dass** die RNA mit der Technik der Reverse-Transkriptase-Kettenreaktion (RT-PCR) amplifiziert wird.

3. Verfahren nach Anspruch 2, **dadurch gekennzeichnet, dass** die benannten RNA durch Gelfärbung, durch die Technik des Radioimmunoassays (RIA), durch den Enzymelinked-Immunosorbent-Assay (ELISA) oder durch Microchip-Tests (Gene-Arrays) analysiert werden.

4. Verfahren nach Anspruch 3, **dadurch gekennzeichnet, dass** die RNA einer Quantifizierung unterworfen werden.

5. Verfahren nach Anspruch 3 oder 4, **dadurch gekennzeichnet, dass** die Quantifizierung mit "TaqMan" oder mit "LightCycler"-Kapillaren ausgeführt wird.

## Claims

1. Method for the diagnosis and/or the monitoring of the evolution of cancers including the analysis of RNA of the telomerase enzyme present in the plasma or serum of the blood, **characterized by** the fact that the RNA is extracted from plasma or serum, purified and amplified, and then that the RNA hTERT coding for the catalytic part of the enzyme or the RNA TEP1 coding for the associate protein is analyzed.

2. Method according to claim 1, **characterized by** the fact that the RNA are amplified by reversed transcriptase chain reaction (RT-PCR).

3. Method according to claim 2, **characterized by** the fact that the RNA are analyzed by gel coloration, by radioactive immunological technique (RIA), by enzyme linked immunosorbant test (ELISA) or by a microchip test (gene array).

4. Method according to claim 3, **characterized by** the fact that the RNA are submitted to a quantification.

5. Method according to claim 3 or 4, **characterized by** the fact that the quantification is carried out on « Taq Man » or on capillaries « Light Cycler ».
